(19) Europäisches Patentamt
European Patent Office
Office européen des brevets

(11) Veröffentlichungsnummer: **0 095 626**
**B1**

(12) # EUROPÄISCHE PATENTSCHRIFT

(45) Veröffentlichungstag der Patentschrift: **23.03.88**

(21) Anmeldenummer: **83104699.0**

(22) Anmeldetag: **13.05.83**

(51) Int. Cl.⁴: **C 07 D 211/14,**
C 07 D 405/04,
C 07 D 211/34,
C 07 D 211/62,
C 07 D 211/50,
C 07 D 211/46,
C 07 D 211/38, C 09 K 19/34,
G 01 R 31/12

(54) **Piperidinderivate.**

(30) Priorität: **28.05.82 DE 3220155**

(43) Veröffentlichungstag der Anmeldung:
**07.12.83 Patentblatt 83/49**

(45) Bekanntmachung des Hinweises auf die Patenterteilung:
**23.03.88 Patentblatt 88/12**

(84) Benannte Vertragsstaaten:
**CH DE FR GB LI**

(56) Entgegenhaltungen:
**EP-A-0 019 665**
**EP-A-0 023 730**
**EP-A-0 056 501**
**DE-A-2 702 598**
**DE-A-3 022 818**

(73) Patentinhaber: **Merck Patent Gesellschaft mit beschränkter Haftung**
**Frankfurter Strasse 250**
**D-6100 Darmstadt (DE)**

(72) Erfinder: **Sucrow, Wolfgang, Prof. Dr.**
**Hüfferweg 13**
**D-4790 Paderborn (DE)**
Erfinder: **Schatull, Wolfgang**
**Am Hilligenbusch 47**
**D-4790 Paderborn (DE)**
Erfinder: **Fuss, Peter, Dr.**
**Im Seegraben 5**
**D-6109 Mühltal-Traisa (DE)**

**EP 0 095 626 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue Piperidinderivate der Formel I

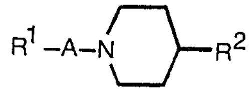

I

worin

$R^1$ und $R^2$ jeweils Alkyl oder Alkoxy mit jeweils 1—10 C-Atomen, F, Cl, Br, CN, der Rest $R^1$ auch H, und A 1,4-Cyclohexylen oder 1,3-Dioxan-2,5-diyl bedeuten,

sowie deren Säureadditionssalze.

Diese Substanzen können wie ähnliche, z.B. aus der DE—OS 27 02 598 bekannte Verbindungen als Komponenten flüssigkristalliner Dielektrika verwendet werden, insbesondere für Displays, die auf dem Prinzip der verdrillten Zelle beruhen.

Weitere ähnliche Verbindungen, die neben einem Piperidinring jedoch mindestens zwei zusätzliche carbocyclische Ringe enthalten, sind aus der EP—A—0 023 730 und der EP—A—0 019 665 bekannt.

Der Erfindung lag die Aufgabe zugrunde, neue stabile flüssigkristalline oder mesogene Verbindungen aufzufinden, die als Komponenten flüssigkristalliner Dielektrika geeignet sind, insbesondere für nematische Phasen mit breitem nematischem Bereich, niedriger Viskosität und/oder kleiner Doppelbrechung. Diese Aufgabe wurde mit der Bereitstellung der Verbindungen der Formel I gelöst.

Es wurde gefunden, daß die Verbindungen der Formel I als Komponenten flüssigkristalliner Dielektrika vorzüglich geeignet sind. Insbesondere sind mit ihrer Hilfe stabile flüssigkristalline Phasen mit breitem nematischem Bereich, niedriger Viskosität und kleiner Doppelbrechung herstellbar.

Die Verbindungen der Formel I zeichnen sich durch kleine Doppelbrechung (niedrige optische Anisotropie) sowie durch ihren polaren Charakter aus. Wegen der niedrigen Doppelbrechung beiten sie Vorteile insbesondere im Fall der verdrillten Zelle mit einem besonders winkelunabhängigen Kontrast (vgl. DE—OS 30 22 818). Aufgrund des polaren Charakters sind sie als Lösungsmittel bzw. als Lösungsvermittler, z.B. für dichroitische Farbstoffe ("host" für "guest-host-Gemische") besonders geeignet. Als Basen bilden sie mit Säuren form-anisotrope Salze; diese rufen eine anisotrope (richtungsabhängige) Leitfähigkeit hervor, die für die Erzeugung einer dynamischen Streuung mit hohem Kontrast günstig ist.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die Viskosität oder die Doppelbrechung eines solchen Dielektrikums zu senken. Die Verbindungen der Formel I, insbesondere diejenigen, die Cyclohexan- oder Dioxanringe mit cisständigen Substituenten enthalten, sowie diejenigen, worin $R^1$ und/order $R^2$ H, Cl, Br oder CN bedeuten, eigenen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch sind sie sehr stabil.

Gegenstand der Erfindung sind somit die Verbindungen der Formel I sowie ein Verfahren zu ihrer Herstellung, dadurch gekennzeichnet, daß man eine sonst der Formel I entsprechende Verbindung, die jedoch an Stelle von Wasserstoffatomen eine oder mehrere zusätzliche reduzierbare Gruppe(n) und/oder C-C-Bindungen enthält, mit einem Reduktionsmittel behandelt,

oder daß man zur Herstellung von Nitrilen der Formel I $R^1$ und/oder $R^2$ = CN) ein entsprechendes Carbonsäureamid dehydratisiert,

oder daß man zur Herstellung von Dioxanderivaten der Formel I (A = 1,3-Dioxan-2,5-diyl) eine entsprechende Formylverbindung oder eines ihrer reaktionsfähigen Derivate mit einem entsprechenden 1,3-Diol umsetzt,

und daß man gegebenenfalls eine Base der Formel I durch Behandeln mit einer Säure in eines ihrer Säure-additionssalze umwandelt,

oder daß man gegebenenfalls aus einem Säureadditionssalz einer Verbindung der Formel I diese durch Behandlung mit einer Base freisetzt.

Weiterhin ist Gegenstand der Erfindung die Verwendung der Verbindungen der Formel I als Komponenten flüssigkristalliner Dielektrika. Gegenstand der Erfindung sind weiterhin flüssigkristalline Dielektrika mit einem Gehalt an mindestens einer Verbindung der Formel I sowie elektrooptische Anzeigeelemente, die derartige Dielektrika enthalten.

Vor- und nachstehend haben $R^1$, $R^2$ und A die angegebene Bedeutung, sofern nicht ausdrücklich etwas anderes vermerkt ist. Der Einfachheit halber bedeuten im folgenden "Cy" 1,4-Cyclohexylen, "Dio" 1,3-Dioxan-2,5-diyl und "Pip" Piperidin-1,4-diyl. Die Formel I kann dann auch in der Form $R^1$-A-Pip-$R^2$ geschrieben werden.

Die Verbindungen der Formel I umfassen beispielsweise die bevorzugten Verbindungen der Formel Ia,

$R^1$-Cy-Pip-$R^2$ Ia

weiterhin die Verbindungen der Formel Ib

$R^1$-Dio-Pip-$R^2$ Ib,

wobei diese Formel die 2-$R^1$-5-(Pip-$R^2$)-1,3-dioxane und die 2-(Pip-$R^2$)-5-$R^1$-1,3-dioxane umschließt.

In den Verbindungen der Formel I sowie Ia und Ib sind diejenigen Stereoisomeren bevorzugt, worin die Substituenten an den 1,4-Cyclohexylenresten jeweils in trans-Stellung zueinander stehen.

In den Verbindungen der Formel I sowie Ia und Ib können die Alkyl- bzw. Alkoxyreste geradkettig oder verzweigt sein. Vorzugsweise sind sie geradkettig, haben 2, 3, 4, 5 oder 6 C-Atome und bedeuten demnach bevorzugt Ethyl, Propyl, Butyl, Pentyl, Hexyl, Ethoxy, Propoxy, Butoxy, Pentoxy oder Hexoxy, ferner Methyl, Heptyl, Octyl, Nonyl, Decyl, Methoxy, Heptoxy, Octoxy, Nonoxy oder Decoxy.

Verbindungen der Formeln I sowie Ia und Ib mit verzweigten Flügelgruppen $R^1$ bzw. $R^2$ können gelegentlich wegen einer besseren Löslichkeit in den üblichen flüssigkristallinen Basismaterialien von Bedeutung sein, insbesonder aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Heptyl (= 1-Methylhexyl), 2-Octyl (= 1-Methylheptyl), 2-Ethylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Im einzelnen bedeuten $R^1$ und $R^2$ bevorzugt Alkyl, Alkoxy oder CN. Unter den Verbindungen der Formeln I sowie Ia und Ib sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln Ic bis If:

NC-Cy-Pip-$R^2$ Ic

NC-Cy-Pip-Alkyl Id

Alkyl-Cy-Pip-Alkyl Ie

Alkoxy-Cy-Pip-Alkyl If

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel Ia werden bevorzugt hergestellt, indem man ein Enamin der Formel II

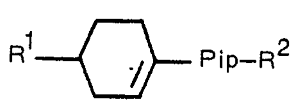 II

reduziert.

Die Enamine der Formel II sind beispielsweise erhältlich durch Umsetzung von 4-$R^1$-Cyclohexanonen mit 4-$R^2$-Piperidinen.

Die Reduktion der Enamine der Formel II erfolgt zweckmäßig durch katalytische Hydrierung bei Temperaturen zwischen etwa 0° und etwa 200° sowie Drucken zwichen etwa 1 und etwa 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan.

Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontiumcarbonat) oder in feinverteilter Form (z.B. Pt-Mohr) eingesetzt werden können.

Nach der Reduktionsstufe kann es zweckmäßig sein, ein gegebenenfalls erhaltenes Stereoisomerengemisch zu trennen, z.B. durch fraktionierte Kristallisation der Hydrochloride. In der Regel sind die Hydrochloride der trans-Isomeren schwerer löslich.

Zur Herstellung von Nitrilen der Formel I, insbesondere solchen der Formeln Ic und Id, können entsprechende Säureamide, z.B. solche der Formeln $H_2N$-CO-A-Pip-$R^2$ oder $R^1$-A-Pip-$CONH_2$, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $POCl_3$, $PCl_5$, $SO_2Cl_2$, $COCl_2$, ferner $P_2O_5$, $AlCl_3$ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0 und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Dioxanderivate der Formel Ib werden zweckmäßig durch Reaktion einer entsprechenden Formylverbindung der Formel $R^1$-CHO bzw. OCH-Pip-$R^2$ (oder eines ihrer reaktionsfähigen Derivate) mit einem entsprechenden 1,3-Diol der Formel $(HOCH_2)_2CH$-Pip-$R^2$ bzw. $R^1CH(CH_2OH)_2$ (oder einem seiner reaktionsfähigen Derivate) hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen etwa 20 und etwa 150, vorzugsweise zwischen 80 und 120°. Als reaktionsfähige Derivate der Ausgangsstoffe eignen sich in erster Linie Acetale der Formeln $R^1$-$CH(OR^3)_2$, $(R^3O)_2CH$-Pip-$R^2$, $R^4$-$CH(OCH_2)_2CH$-Pip-$R^2$ bzw. $R^1$-$CH(CH_2O)_2CH$-$R^5$, worin $R^3$ Alkyl mit 1—4 C-Atomen, zwei Reste $R^3$ zusammen auch Alkylen mit 2 oder 3 C-Atomen und $R^4$ H, Alkyl mit 1—4 C-Atomen oder Phenyl bedeuten.

Die genannten Formylverbindungen und 1,3-Diole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde der Formel $R^1$-CHO durch Oxydation entsprechender Alkohole oder durch Reduktion entsprechender Carbonsäuren oder ihrer Derivate, die Formylpiperidine der Formel OCH-Pip-$R^2$ durch Formylierung entsprechender Piperidine, die Diole durch Reduktion entsprechender Diester der Formeln $(AlkylOOC)_2CH$-Pip-$R^2$ bzw. $R^1CH(COOAlkyl)_2$ erhältlich.

Eine Base der Formel I kann mit einer Säure in das zugehörige Säureadditionssalz übergeführt werden. Für diese Umsetzung können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, naphthalin-mono- und disulfonsäuren, Laurylschwefelsäure.

Umgekehrt ist es möglich, aus einem Säureadditionssalz einer Verbindung der Formel I die Base der Formel I durch Behandeln mit einer Base freizusetzen, z.B. mit einer starken anorganischen Base wie KOH oder NaOH.

Die erfindungsgemäßen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Biscyclohexylbiphenyle, Phenyl- oder Cyclohexylpyrimidine, Phenyl- oder Cyclohexyldioxane, gegebenenfalls halogenierte Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel III charakterisieren,

$$R^5\text{-D-G-E-}R^6 \qquad \text{III}$$

worin D und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

—CH = CH— —N(O) = N—

—CH = CX— —CH = N(O)—

| | |
|---|---|
| —C ≡ C— | —$CH_2$—$CH_2$— |
| —CO—O— | —$CH_2$—O— |
| —CO—S— | —$CH_2$—S— |
| —CH = N— | —COO-Phe-COO— |

oder eine C-C-Einfachbindung, X Halogen, vorzugsweise Chlor, oder —CN, und $R^5$ und $R^6$ Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind $R^5$ und $R^6$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemäßen Dielektrika enthalten etwa 0,1 bis 60, vorzugsweise 5 bis 40%, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur auführlich beschrieben. Beispielsweise können Leitsalze, vorzugsweise Ethyl-dimethyl-dodecylammonium-4-hexyloxybenzoat, Tetrabutylammonium-tetraphenylboranat oder Komplexsalze von Kronenethern (vergl. z.B. I. Haller et al., Mol. Cryst. Liq. Cryst. Band *24*, Seiten 249—258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind zum Beispiel in den DE—OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben.

Beispiel 1

Man hydriert eine Lösung von 5 g 1-(4-Methoxycarbonylcyclohexen-1-yl)-4-pentylpiperidin (Kp. 171°/0,4 Torr; erhältlich aus Cyclohexanon-4-carbonsäuremethylester und 4-Pentylpiperidin in Toluol in Gegenwart von p-Toluolsulfonsäure) in 50 ml THF mit 0,5 g 10%igem Pd-C unter 2 bar $H_2$, filtriert, dampft ein und erhält 1-(trans-4-Methoxycarbonylcyclohexyl)-4-pentylpiperidin, F. 5°, Kp. 177°/0,27 mbar. (Ausgangsstoff für Beispiel 2).

Analog erhält man durch Hydrierung der entsprechenden Enamine:

1-Cyclohexyl-4-pentylpiperidin
1-(trans-4-Methylcyclohexyl)-4-pentylpiperidin
1-(trans-4-Propylcyclohexyl)-4-propylpiperidin
1-(trans-4-Propylcyclohexyl)-4-butylpiperidin
1-(trans-4-Propylcyclohexyl)-4-pentylpiperidin
1-(trans-4-Butylcyclohexyl)-4-propylpiperidin
1-(trans-4-Butylcyclohexyl)-4-butylpiperidin
1-(trans-4-Butylcyclohexyl)-4-pentylpiperidin
1-(trans-4-Pentylcyclohexyl)-4-propylpiperidin
1-(trans-4-Pentylcyclohexyl)-4-butylpiperidin
1-(trans-4-Pentylcyclohexyl)-4-pentylpiperidin
1-(trans-4-Decylcyclohexyl)-4-pentylpiperidin
1-(trans-4-Methoxycyclohexyl)-4-pentylpiperidin
1-(trans-4-Propoxycyclohexyl)-4-propylpiperidin
1-(trans-4-Propoxycyclohexyl)-4-butylpiperidin
1-(trans-4-Propoxycyclohexyl)-4-pentylpiperidin
1-(trans-4-Butoxycyclohexyl)-4-propylpiperidin
1-(trans-4-Butoxycyclohexyl)-4-butylpiperidin
1-(trans-4-Butoxycyclohexyl)-4-pentylpiperidin
1-(trans-4-Pentoxycyclohexyl)-4-propylpiperidin
1-(trans-4-Pentoxycyclohexyl)-4-butylpiperidin
1-(trans-4-Pentoxycyclohexyl)-4-pentylpiperidin
1-(trans-4-Decoxycyclohexyl)-4-pentylpiperidin
1-(trans-4-Fluorcyclohexyl)-4-propylpiperidin

1-(trans-4-Fluorcyclohexyl)-4-butylpiperidin
1-(trans-4-Fluorcyclohexyl)-4-pentylpiperidin
1-(trans-4-Chlorcyclohexyl)-4-pentylpiperidin
1-(trans-4-Bromcyclohexyl)-4-pentylpiperidin.

Beispiel 2

Man rührt eine Lösung von 100 mg 1-(4-Carbamoylcyclohexyl)-4-pentyl-piperidin [F. 186°; erhältlich durch Verseifung von 1-(trans-4-Methoxycarbonylcyclohexyl)-4-pentylpiperidin mit methanolischem KOH zur Carbonsäure (F. 208°), Überführung in das Säurechlorid mit $SOCl_2$ und Reaktion mit wässeriger $NH_3$-Lösung] und 1 ml $POCl_3$ in 5 ml Pyridin 15 Std. bei 20°, dampft ein und kristallisiert das erhaltene 1-(trans-4-Cyancyclohexyl)-4-pentylpiperidin-hydrochlorid aus Ethylacetat um. F. 260° (Zers.). Freie Base, F. 56,6°, K. 64,8°.

Analog erhält man durch Dehydratisierung der entsprechenden Amide:
1-(trans-4-Cyancyclohexyl)-4-fluorpiperidin
1-(trans-4-Cyancyclohexyl)-4-methylpiperidin
1-(trans-4-Cyancyclohexyl)-4-ethylpiperidin
1-(trans-4-Cyancyclohexyl)-4-propylpiperidin
1-(trans-4-Cyancyclohexyl)-4-butylpiperidin
1-(trans-4-Cyancyclohexyl)-4-hexylpiperidin
1-(trans-4-Cyancyclohexyl)-4-heptylpiperidin
1-(trans-4-Cyancyclohexyl)-4-octylpiperidin
1-(trans-4-Cyancyclohexyl)-4-nonylpiperidin
1-(trans-4-Cyancyclohexyl)-4-decylpiperidin
1-(trans-4-Cyancyclohexyl)-4-methoxypiperidin
1-(trans-4-Cyancyclohexyl)-4-ethoxypiperidin
1-(trans-4-Cyancyclohexyl)-4-propoxypiperidin
1-(trans-4-Cyancyclohexyl)-4-butoxypiperidin
1-(trans-4-Cyancyclohexyl)-4-pentoxypiperidin
1-(trans-4-Cyancyclohexyl)-4-decoxypiperidin.

Beispiel 3

Ein Gemisch von 2,29 g 1-Dimethoxymethyl-4-pentylpiperidin (erhältlich durch Formylierung von 4-Pentylpiperidin und anschließende Reaktion mit Dimethylsulfat und Na-Methylat/Methanol), 1,46 g 2-Pentylpropan-1,3-diol, 0,01 g p-Toluolsulfonsäure und 20 ml Toluol wird 3 Std. gekocht, wobei man das gebildete Methanol abdestilliert, abgekühlt, mit Wasser gewaschen und eingedampft. Man erhält 2-(4-Pentyl-1-piperidyl)-5-pentyl-1,3-dioxan. F. 46—51°.

Analog erhält man durch Umsetzung der entsprechenden 1-Dimethoxymethylpiperidine mit den entsprechenden Diolen:
2-(4-Propyl-1-piperidyl)-5-propyl-1,3-dioxan
2-(4-Butyl-1-piperidyl)-5-propyl-1,3-dioxan
2-(4-Pentyl-1-piperidyl)-5-propyl-1,3-dioxan
2-(4-Propyl-1-piperidyl)-5-butyl-1,3-dioxan
2-(4-Butyl-1-piperidyl)-5-butyl-1,3-dioxan
2-(4-Pentyl-1-piperidyl)-5-butyl-1,3-dioxan
2-(4-Propyl-1-piperidyl)-5-pentyl-1,3-dioxan
2-(4-Butyl-1-piperidyl)-5-pentyl-1,3-dioxan.

Beispiel 4

Ein Gemisch von 1 g Hexanal, 2,29 g 1-(1,3-Dihydroxy-2-propyl)-4-pentylpiperidin [erhältlich durch Reaktion von Brommalonsäurediethylester mit 4-Pentylpiperidin zu 1-Bis-(ethoxycarbonyl)-methyl-4-pentyl-piperidin und nachfolgende Reduktion mit $LiAlH_4$], 0,01 g p-Toluolsulfonsäure und 15 ml Toluol wird am Wasserabscheider 3 Std. gekocht, abgekühlt, mit Wasser gewaschen und eingedampft. Man erhält 2-Pentyl-5-(4-pentyl-1-piperidyl)-1,3-dioxan.

Analog erhält man durch Umsetzung der entsprechenden Aldehyde mit den entsprechenden Diolen:
2-Propyl-5-(4-propyl-1-piperidyl)-1,3-dioxan
2-Butyl-5-(4-propyl-1-piperidyl)-1,3-dioxan
2-Pentyl-5-(4-propyl-1-piperidyl)-1,3-dioxan
2-Propyl-5-(4-butyl-1-piperidyl)-1,3-dioxan
2-Butyl-5-(4-butyl-1-piperidyl)-1,3-dioxan
2-Pentyl-5-(4-butyl-1-piperidyl)-1,3-dioxan
2-Propyl-5-(4-pentyl-1-piperidyl)-1,3-dioxan
2-Butyl-5-(4-pentyl-1-piperidyl)-1,3-dioxan.

Es folgen Beispiele für erfindungsgemäße Dielektrika mit einem Gehalt an mindestens einer Verbindung der Formel I:

Beispiel A

Ein Gemisch aus
23% 1-(trans-4-Cyancyclohexyl)-4-pentylpiperidin
28% trans,trans-4-Ethylcyclohexylcyclohexan-4'-carbonitril
19% trans,trans-4-Propylcyclohexylcyclohexan-4'-carbonitril
30% trans,trans-4-Heptylcyclohexylcyclohexan-4'-carbonitril
zeigt F. 3°, K. 68°.

Beispiel B

Ein Gemisch aus
15% 1-(trans-4-Cyancyclohexyl)-4-propylpiperidin
15% 1-(trans-4-Cyancyclohexyl)-4-pentylpiperidin
20% trans,trans-4-Butylcyclohexylcyclohexan-4'-carbonitril
18% trans-1-p-Ethoxyphenyl-4-propylcyclohexan
15% p-(trans-4-Propylcyclohexyl)-benzonitril
7% trans,trans-4-Butylcyclohexylcyclohexan-4'-carbonsäure-trans-4-propyl-cyclohexylester
10% trans-4-Pentylcyclohexancarbonsäure-p-(trans-4-propylcyclohexyl)-phenylester
zeigt F. −7°, K. 57°.

Beispiel C

Ein Gemisch aus
15% 1-(trans-4-Cyancyclohexyl)-4-propylpiperidin
15% 1-(trans-4-Cyancyclohexyl)-4-pentylpiperidin
25% trans,trans-4-Pentylcyclohexylcyclohexan-4'-carbonitril
25% trans,trans-4-Heptylcyclohexylcyclohexan-4'-carbonitril
10% trans-1-p-Butoxyphenyl-4-propylcyclohexan
8% 4,4'-Bis-(trans-4-pentylcyclohexyl)-biphenyl
2% 1,5-Diamino-4,8-dihydroxy-3-p-methoxyphenyl-anthrachinon
zeigt K. 82°.

**Patentansprüche**

1. Piperidinderivate der Formel I

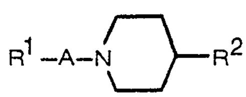

I

worin
$R^1$ und $R^2$ jeweils Alkyl oder Alkoxy mit jeweils 1—10 C-Atomen, F, Cl, Br, CN, der Rest $R^1$ auch H, und
A 1,4-Cyclohexylen oder 1,3-Dioxan-2,5-diyl bedeuten,
sowie deren Säureadditionssalze.

2. Verfahren zur Herstellung von Piperidinderivaten der Formel I nach Anspruch 1, dadurch gekennzeichnet, daß man eine sonst der Formel I entsprechende Verbindung, die jedoch an Stelle von Wasserstoffatomen eine oder mehrere zusätzliche reduzierbare Gruppen und/oder C-C-Bindungen enthält, mit einem reduzierenden Mittel behandelt

oder daß man zur Herstellung von Nitrilen der Formel I $R^1$ und/oder $R^2$ = CN) ein entsprechendes Carbonsäureamid dehydratisiert,

oder daß man zur Herstellung von Dioxanderivaten der Formel I (A = 1,3-Dioxan-2,5-diyl) eine entsprechende Formylverbindung oder eines ihrer reaktionsfähigen Derivate mit einem entsprechenden 1,3-Diol umsetzt

und daß man gegebenenfalls eine Base der Formel I durch Behandeln mit einer Säure in eines ihrer Säureadditionssalze umwandelt,

oder daß man gegebenenfalls aus einem Säureadditionssalz einer Verbindung der Formel I diese durch Behandlung mit einer Base freisetzt.

3. Verwendung der Verbindungen der Formel I nach Anspruch 1 als Komponenten flüssigkristalliner Dielektrika für elektrooptische Anzeigeelemente.

4. Flüssigkristallines Dielektrikum für elektrooptische Anzeigeelemente mit mindestens zwei flüssigkristallinen Komponenten, dadurch gekennzeichnet, daß mindestens eine Komponente eine Verbindung der Formel I nach Anspruch 1 ist.

5. Elektrooptisches Anzeigeelement, dadurch gekennzeichnet, daß es ein Dielektrikum nach Anspruch 4 enthält.

**Revendications**

1. Dérivés de la pipéridine de formule I

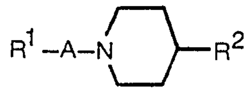

I

dans laquelle

$R^1$ et $R^2$ représentent chacun un groupe alkyle ou alcoxy contenant chacun 1 à 10 atomes de carbone, F, Cl, Br, CN, le symbole $R^1$ pouvant également représenter l'hydrogène, et

A représente un groupe 1,4-cyclohexylène ou 1,3-dioxanne-2,5-diyle, et leurs sels formés par addition avec des acides.

2. Procédé de préparation des dérivés de la pipéridine de formule I selon la revendication 1, caractérisé en ce que l'on traite par un agent réducteur un composé répondant par ailleurs à la formule I mais portant à la place d'atomes d'hydrogène un ou plusieurs autres groupes réductibles et/ou liaisons C—C,

ou bien en ce que, pour préparer les nitriles de formule I ($R^1$ et/ou $R^2$ = CN) on déshydrate un carboxamide correspondant,

ou bien en ce que, pour préparer les dérivés du dioxanne de formule I (A = 1,3-dioxanne-2,5-diyle), on fait réagir un composé formylé correspondant ou l'un de ses dérivés réactifs avec un 1,3-diol correspondant,

et le cas échéant, on convertit une base de formule I, par traitement à l'aide d'un acide, en un sel formé par addition avec un acide,

ou bien encoure, le cas échéant, on libère un composé de formule I à partir d'un sel formé par addition avec un acide en traitant par une base.

3. Utilisation de composés de formule I selon la revendication 1 en tant que composants de diélectriques à cristaux liquides pour éléments d'affichage électro-optiques.

4. Diélectrique à cristaux liquides pour éléments d'affichage électro-optiques contenant au moins deux composants à cristaux liquides, caractérisé en ce que l'un au moins des composants est un composé de formule I selon la revendication 1.

5. Elément d'affichage électro-optique, caractérisé en ce qu'il contient un diélectrique selon la revendication 4.

**Claims**

1. Piperidine derivatives of the formula I

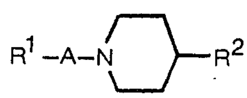

I

in which

$R^1$ and $R^2$ each are alkyl or alkoxy each having 1 to 10 C atoms, F, Cl, Br or CN, and the radical $R^1$ even H, and

A is 1,4-cyclohexylene or 1,3-dioxane-2,5-diyl, and their acid addition salts.

2. Process for preparing piperidine derivatives of the formula I according to Claim 1, characterised in that a compound which otherwise has the formula I but which in place of hydrogen atoms contains one or more additional reducible groups and/or C—C bonds is treated with a reducing agent,

or that to prepare nitriles of the formula I ($R^1$ and/or $R^2$ = CN) a corresponding carboxamide is dehydrated,

or that to prepare dioxane derivatives of the formula I (A = 1,3-dioxane-2,5-diyl) a corresponding formyl compound or one of its reactive derivatives is reacted with a corresponding 1,3-diol,

and that, if desired, a base of the formula I is treated with an acid and thus converted into one of its acid addition salts,

or that, if desired, an acid addition salt of a compound of the formula I is treated with a base to liberate the compound of the formula I.

3. Use of compounds of the formula I according to Claim 1, as components of liquid-crystalline dielectrics for electro-optical display elements.

4. Liquid-crystalline dielectric material for electro-optical display elements which contains at least two liquid-crystalline components, characterised in that at least one component is a compound of the formula I according to Claim 1.

5. Electro-optical display element, characterised in that it contains a dielectric material according to Claim 4.